# EUROPEAN PATENT SPECIFICATION

(11) **EP 0 531 640 B1**
(45) Date of publication and mention of the grant of the patent: **26.11.1997**
(21) Application number: 92110954.2
(22) Date of filing: 29.06.1992
(51) Int. Cl.: C07H 7/02, C12P 19/02, A23K 1/17, A61K 31/71

(54) **Antibiotic LL-E19020 Gamma**
Antibiotikum LL-E19020 Gamma
Antibiotique LL-E19020 gamma

(30) Priority: 09.09.1991 US 756411; 09.09.1991 US 756938
(43) Date of publication of application: 17.03.1993
(73) Proprietor: F. HOFFMANN-LA ROCHE AG, 4070 Basel (CH)
(72) Inventor: Carter, Guy Thomas, Suffern, NY 10901 (US); Williams, David R., Stony Point, NY 10980 (US); Korshalla, Joseph D., Pearl River, NY 10965 (US); Hart, Ian Christopher, Pennington, NJ 08534 (US)
(74) Representative: Kellenberger, Marcus, Dr.

(56) References cited:
- US-A- 4 704 276
- US-A- 4 705 688
- JOURNAL OF CHROMATOGRAPHY, vol. 483, 1989, Amsterdam, NL, D.R. WILLIAMS et al., pp. 381-390

## Description

### BACKGROUND OF THE INVENTION

### 1. FIELD OF THE INVENTION

The invention relates to a new antibiotic designated LL-E19020 Gamma, to its production by fermentation and to a process for its recovery and purification.

### 2. DESCRIPTION OF THE PRIOR ART

Antibiotics LL-E19020 Alpha and LL-E19020 Beta are disclosed in U.S. Patent 4,705,688, The Journal Of Antibiotics, 41(10), 1511 - 1514 (1988) and The Journal Of Antibiotics, 42(10), 1489 - 1493 (1989). Antibiotic LL-E19020 Alpha has a phenylacetate ester group attached at C-23 and has the structure:

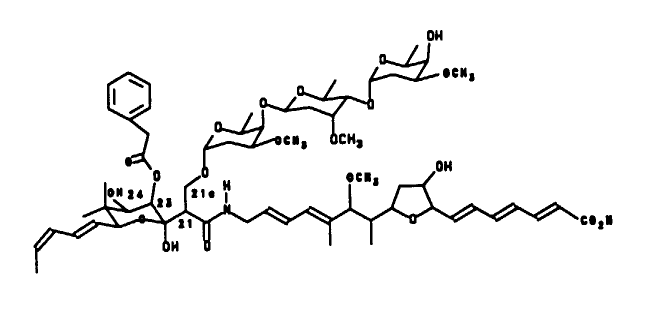

Antibiotic LL-E19020 Beta has a phenylacetate ester group attached at C-24 and has the structure:

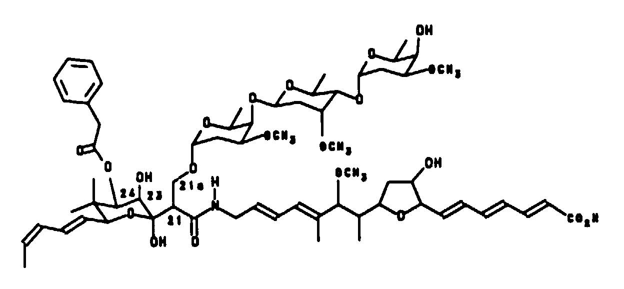

A process for purification of the antibiotic LL-E19020 Alpha by reversed phase HPLC purification is described in Journal Of Chromatography, 484, 381-390(1989). Antibiotics LL-E19020 Alpha and LL-E 19020 Beta are also useful for increasing the growth rate of meat producing animals and for treating respiratory disease, fowl cholera and necrotic enteritis as described in U.S. 4,704,276 and U.S. 4,968,493.

A related family of compounds, the phenelfamycins, is reported in The Journal Of Antibiotics, 41(10), 1293 - 1299 (1988); The Journal Of Antibiotics, 41(10), 1300 - 1315 (1988); The Journal Of Antibiotics, 39(10), 1361 - 1367 (1986); The Journal Of Antibiotics, 42(1), 94 - 101 (1989); Antimicrobiol Agents and Chemotherapy, 33(3), 322 - 325 (1989); and Program and Abstracts Of The 27th Interscience Conference on Antimicrobial Agents Chemotherapy, No. 995, p 270, New York, October 4 - 7 1987.

### SUMMARY OF THE INVENTION

A new antibiotic designated LL-E19020 Gamma has now been found. The structure of the new antibiotic LL-E19020 Gamma is: 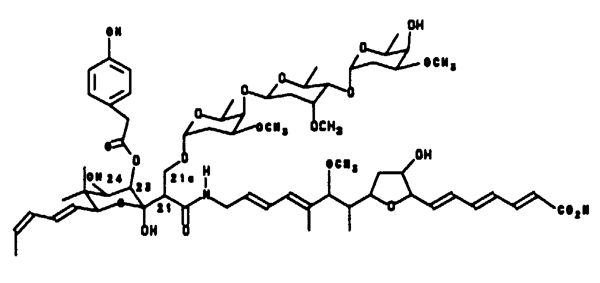 As can be determined from the above structure antibiotic LL-E19020 Gamma differs from the previously known antibiotics LL-E19020 Alpha and LL-E19020 Beta in that LL-E19020 Gamma has a 4-hydroxyphenylacetate ester group attached at C-23. The physico chemical characteristics of LL-E19020 Gamma are as follows:
1. Molecular weight: 1241 (FABMS=M/Z 1264 corresponding to [M+Na]+)
2. Molecular formula: C₆₅H₉₅NO₂₂ microanalysis found(calc): C62.22(62.85); H7.77(7.66); N 0.92(1.13).
HRFABMS: (M+K)+=M/Z 1280.5983 (calc. 1280.5983)
3. Specific optical rotation: [α]_{D} 26^{o} = -7^{o} (1.001,MeOH)
4. Ultraviolet Absorption Spectrum as shown in Figure I.
UV absorption [MeOH]λ max (ε): 231 nm (58,600); 287 nm (39,300).
5. IR absorption spectrum as shown in Figure II. IR absorption spectrum [KBr] ν max: 3411br, 2974, 2934, 2828, 1735, 1716sh, 1700, 1652, 1647, 1617, 1455, 1367, 1222, 1098, 1023 cm⁻¹.
6. Proton ¹H NMR[CDCl₃]: Spectrum (300 MHz) as shown in Figure III.
7. Carbon 13 ¹³C NMR[CDCl₃] Spectrum as shown in Figure IV, significant peaks listed below (δ from TMS):

| | | | |
|---|---|---|---|
| 173.4 | 127.5 | 74.64 | 49.77 |
| 171.9 | 126.3 | 74.51 | 41.85 |
| 170.1 | 125.1 | 74.42 | 40.92 |
| 155.7 | 120.7 | 74.07 | 39.91 |
| 145.7 | 115.6(2X) | 72.19 | 39.23 |
| 140.3 | 100.5 | 71.83 | 38.81 |
| 136.9 | 98.90 | 69.10 | 32.98 |
| 134.3 | 97.28 | 67.57 | 30.98 |
| 132.0 | 96.93 | 66.40 | 29.91 |
| 130.6(2X) | 89.16 | 66.07 | 23.75 |
| 130.1 | 83.21 | 63.57 | 18.10 |
| 129.0 | 81.64 | 56.53 | 17.20 |
| 128.5 | 77.63 | 56.10 | 17.00 |
| 128.3 | 77.20 | 55.50 | 14.84 |
| 128.2 | 76.46 | 55.45 | 13.50 |
| 128.1 | 10.97 | 10.18 | |

8. High pressure liquid chromatography (HPLC) retention time of 18.5 minutes using a gradient of acetonitrile in aqueous acetic acid.
9. High pressure liquid chromatography (HPLC) retention time of 19.6 minutes using a gradient of dioxane in aqueous acetic acid.

The new antibiotic LL-E19020 Gamma is formed along with LL-E19020 Alpha and LL-E19020 Beta during cultivation under controlled conditions of a strain of Streptomyces lydicus ssp. tanzanius, NRRL 18036. The new antibiotic LL-E19020 Gamma is separated from LL-E 19020 Alpha and LL-E19020 Beta and subsequently purified by high pressure liquid chromotography (HPLC).

### Brief Description Of The Drawings

Figure I shows the ultraviolet absorption spectrum of LL-E19020 Gamma.

Figure II shows the infrared absorption spectrum of LL-E19020 Gamma.

Figure III shows the proton nuclear magnetic resonance spectrum of LL-E19020 Gamma.

Figure IV shows the carbon-13 nuclear magnetic resonance spectrum of LL-E19020 Gamma.

### Description Of The Preferred Embodiments

The antibiotic LL-E19020 Gamma is produced by fermentation of a strain of Streptomyces lydicus, ssp. tanzanius, NRRL 18036, in an aqueous nutrient medium containing assimilable sources of carbon and nitrogen under submerged aerobic conditions. This microorganism is maintained in the culture collection of the Medical Research Division, American Cyanamid Company, Pearl River, New York as culture number LL-E19020. A viable culture of this new microorganism has been deposited with the Patent Culture Collection Laboratory, Northern Regional Research Center, U.S. Department of Agriculture, Peoria, Illinois 61604, and has been added to its permanent collection. It has been assigned the strain designation NRRL 18036 by said depository.

Culture LL-E19020 produces short spiral spore chains, 10-50 spores long, with occasional longer chains. These tend to coalesce to form dry blackish masses on such ISP media as oatmeal and inorganic salts-starch. The spores have smooth surfaces as assessed by electron microscopy. The strain contains the L isomer of diaminopimelic acid, and may thus be assigned to the genus Streptomyces.

In the ISP tests for utilization of carbohydrates, LL-E19020 shows growth on arabinose, fructose, inositol, mannitol, reffinose, rhamnose, sucrose and xylose. Cellulose is not utilized.

The reactions of LL-E19020 in the Gordon physiological series are compared in the following Table I with those of Streptomyces lydicus ISP 5461 which it most closely resembles morphologically and physiologically.

Because LL-E19020 differs from ISP 5461 in five(5) characteristics (xanthine hydrolysis, decarboxylation of oxalate, acid from erythritol, rhamnose and β-methyl-D-xyloside) it is designated as a subspecies of Streptomyces lydicus.

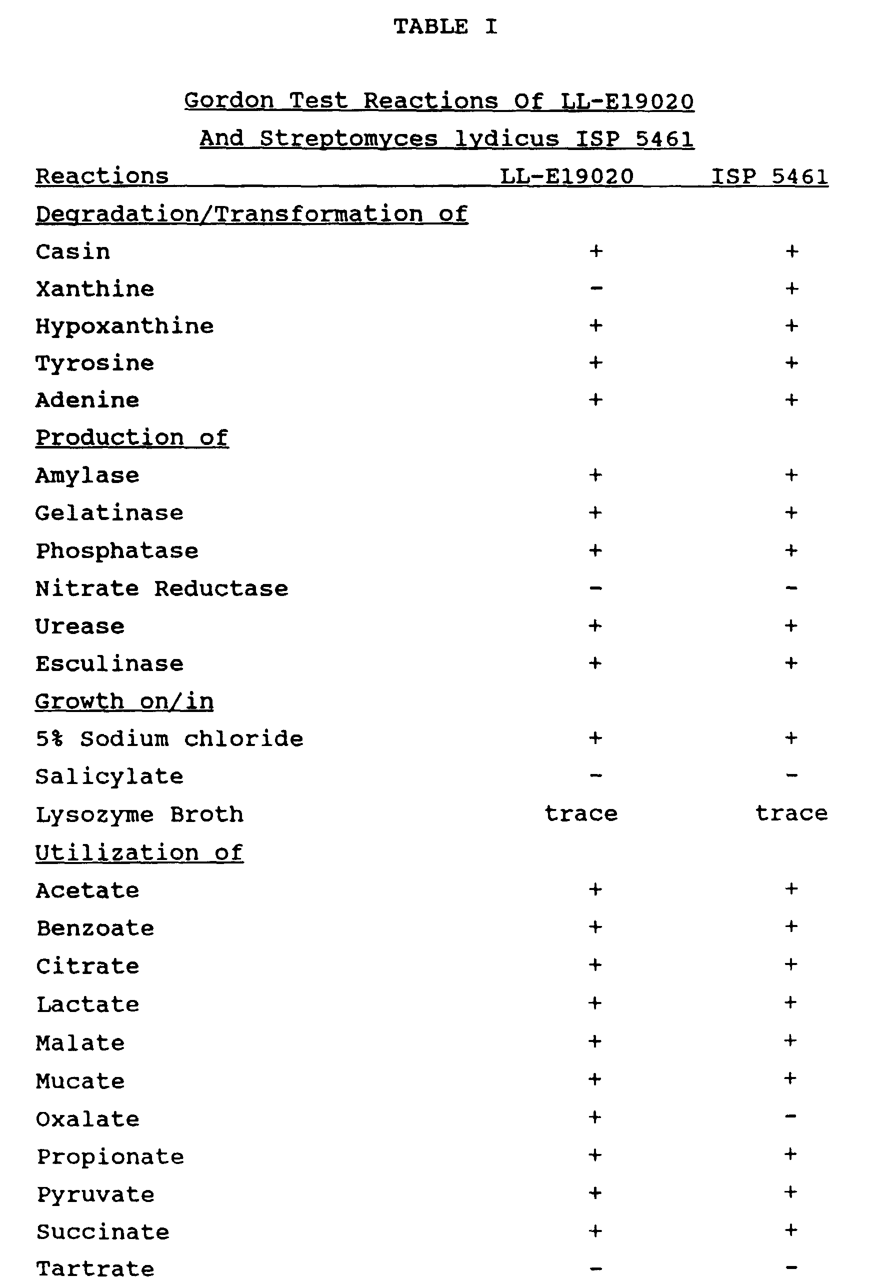

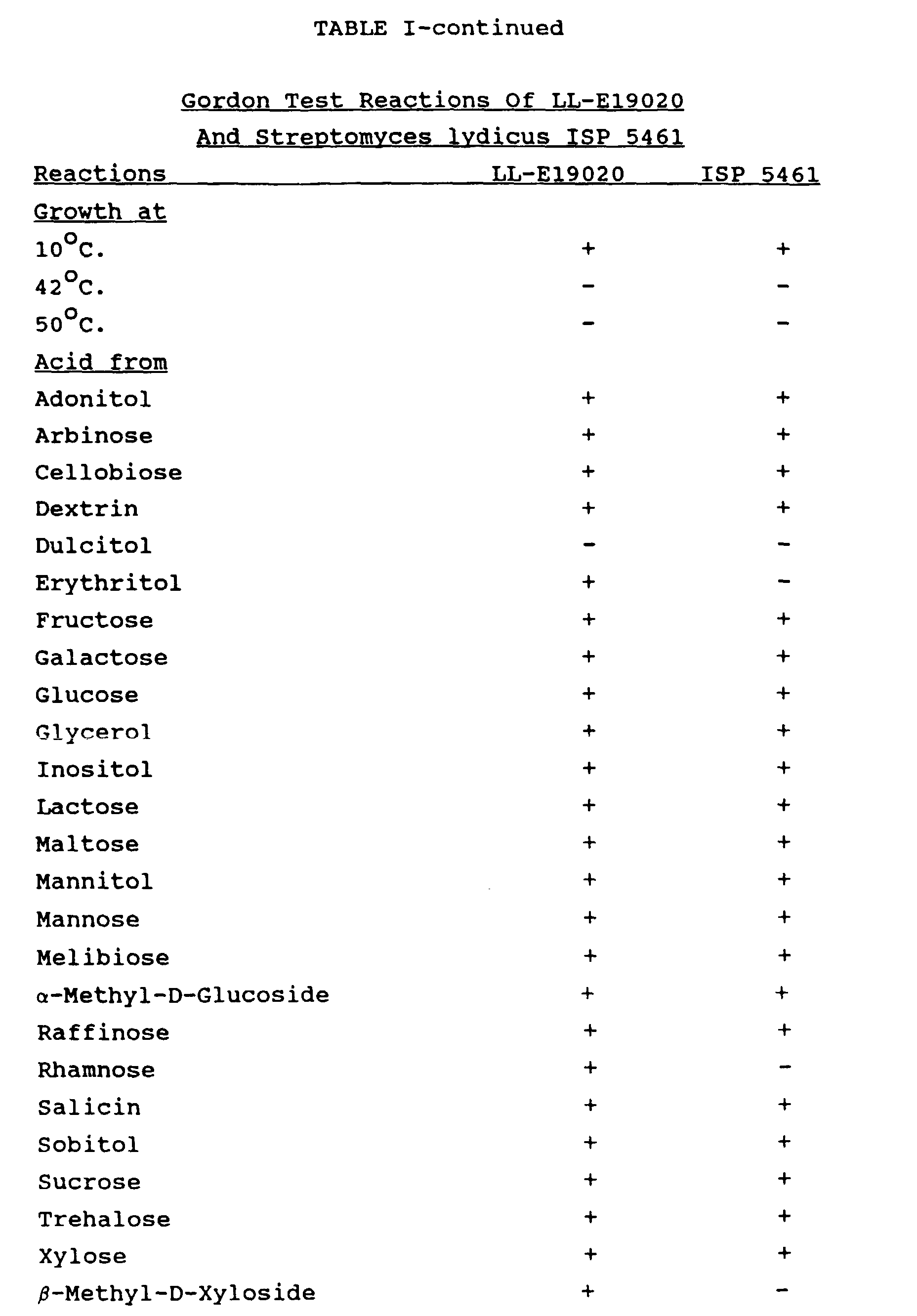

It is to be understood that for the production of these new antibacterial agents the present invention is not limited to this particular organism or to organisms fully answering the above characteristics which are given for illustrative purposes only. In fact, it is desired and intended to include the use of mutants produced from this organism by various means such as exposure of X-radiation, ultraviolet radiation, N'-methyl-N'-nitro-N-nitrosoguanidine, actinophages and the like.

Cultivation of Streptomyces lydicus SSP. tanzanius NRRL 18036 may be carried out in a wide variety of liquid culture media. Media which are useful for the production of LL-E19020 Gamma include an assimilable source of carbon, such as dextrin, sucrose, molasses, glycerol, etc; an assimilable source of nitrogen such as protein, protein hydrolysate, polypeptides, amino acids, corn steep liquor, etc; and inorganic anions and cations, such as potassium, sodium, ammonium, calcium, sulfate, carbonate, phosphate, chloride, etc. Trace elements such as boron, molybdenum, copper, etc., are supplied as impurities of other constituents of the media. Aeration in tanks and bottles is supplied by forcing sterile air through or onto the surface of the fermenting medium. Further agitation in tanks is provided by a mechanical impeller. An antifoam agent such as silicone oil may be added as needed.

The antibiotic LL-E19020 Gamma is recovered from the fermentation broth by extraction of the broth into a solvent such as ethyl acetate, followed by chromatography of the ethyl acetate extracted broth using a high pressure liquid chromatography with a twelve (12) liter reverse phase column (C18 bonded phase 40 micron) using 0.1M ammonium acetate pH 4.3/acetonitrile (1:1) to obtain a mixture of LL-E19020 Alpha and LL-E19020 Gamma. Additional purification of this mixture by high pressure liquid chromatography using a twelve (12) liter reverse-phase column (C18 bonded phase 40 micron) using 0.1M ammonium acetate pH 4.3/acetonitrile (1:1) gives pure LL-E19020 Gamma and pure LL-E19020 Alpha.

### EXAMPLE 1

### INOCULUM PREPARATION

A typical medium used to grow the primary inoculum is prepared according to the following formula:

| | |
|---|---|
| Dextrose | 1.0% |
| Dextrin | 2.0% |
| Yeast extract | 0.5% |
| NZ Amine A | 0.5% |
| Calcium carbonate | 0.1% |
| Antifoam | 0.3% |
| Water qs | 100.0% |
| **NOTE:** NZ Amine A is a pancreatic digest of casein, registered trademark of Scheffield Chemical, Norwich, New York. | |

This medium is sterilized and 100 ml, in a 500 ml flask, is inoculated with Streptomyces lydicus ssp. tanzanius NRRL 18036. The medium is then placed on a rotary shaker and incubated at 28^{o}C for 48 hours providing a primary inoculum. This primary inoculum is the used to inoculate 10 liters of the same sterile medium in a bottle. This culture is grown for 24 hours providing secondary inoculum. This secondary inoculum is then used to inoculate 300 liters of the same sterile medium in a tank. This culture is grown at 30°C for 44 hours with a sterile air flow of 0.66 liters per liter of mash per minute and agitation by an impeller driven at 200 rpm, providing a tertiary inoculum.

### EXAMPLE 2

### FERMENTATION

A fermentation production medium of the following formulation is prepared:

| | |
|---|---|
| Dextrin | 7.0% |
| Dextrose | 0.5% |
| Soy flour | 1.5% |
| Corn Steep liquor | 0.5% |
| Calcium carbonate | 0.5% |
| Silicone antifoam | 0.3% |
| Water qs | 100.0% |

This medium is sterilized and 1500 liters is then inoculated with 150 liters of tertiary inoculum from Example 1. The fermentation is conducted at 28^{o}C with a sterile air flow of 3.3 liters of air per liter of mash per minute and agitation by an impeller driven at 100 rpm for 123 hours, at which time the mash is harvested.

### EXAMPLE 3

A fermentation medium of the following formulation is prepared:

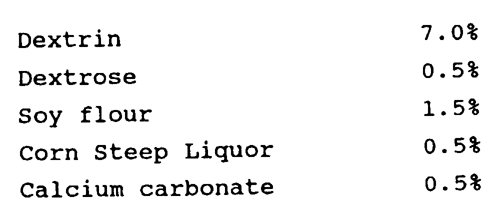

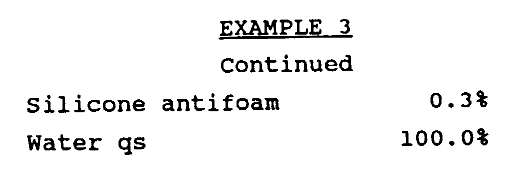

This medium is sterilized and 3000 liters is then inoculated with 300 liters of tertiary inoculum similarly prepared as in Example 1. The fermentation is conducted at 28^{o}C with a sterile air flow of 6.5 liters of air per liter of mash per minute and agitation by an impeller driven at 100 rpm for 89 hours, at which time the mash is harvested.

### EXAMPLE 4

### Isolation and Purification of LL-E19020 Gamma

The harvest mash from two (2) fermentations conducted as described in Example 2 and Example 3, making a total volume of 3200 liters, is diluted with 1600 liters of methyl alcohol and filtered through diatomaceous earth. The filter cake is washed with 320 liters of water and the wash is added to the filtrate giving a total volume of 5000 liters. A 800 liter volume is charged to a still and evaporated to 500 liters. This procedure is repeated until the total volume is reduced to 2950 liters followed by dilution with 1450 liters of ethyl acetate. The lower phase is removed and the upper phase of 900 liters evaporated to 79.5 liters. This concentrate is diluted with 80 liters of ethyl acetate and the lower layer removed. The upper layer is evaporated to give 2.4 liters of a syrup. This crude product is repeatedly decanted with hexane then dissolved in methyl alcohol and applied portion wise to a 12 liter reverse phase column (C18 bonded phase 40 micron). In a typical run, 400 ml of syrup is dissolved in methyl alcohol to give a final volume of 700 ml which is applied to the reverse phase column and eluted with 1:1 0.1M ammonium acetate:acetonitrile at pH 4.3 to afford upon evaporation of the volatiles 38 g of impure LL-E19020 Gamma. Several like runs are completed in this manner and the products combined.

### Purification of LL-E19020 Alpha and LL-E19020 Gamma

A total of 100 g of impure LL-E19020 Gamma is charged to a 12 liter reverse phase column (C18 bonded phase 40 micron) and eluted with 0.1M ammonium acetate buffer pH 4.3/acetonitrile (1:1). Fractions designated F1-F28, each having a volume of 20 liters are collected. Fraction F4 is stirred with 15 liters of methylene chloride for 1 hour. The methylene chloride layer is separated and evaporated to 1 liter, dried with calcium chloride and evaporated to a residue which is dissolved in 75 ml of methyl alcohol and filtered. The filtrate is added, 5 ml at a time, to a 2.2 x 25 cm(10 micron) reverse phase C18 chromatographic column. The column is eluted with 40% acetonitrile in 0.05 M ammonium acetate buffer (pH 4.5) at a flow rate of 9.9 ml/minute. The eluate collected after 2.5 to 3 hours is extracted with ethyl acetate. The organic layer is evaporated to a syrup which is dissolved in t-butanol and freeze dried to afford 160 mg of pure LL-E19020 Gamma.

### ANALYTICAL HIGH PRESSURE LIQUID CHROMATOGRAPHY (HPLC)

The LL-E19020 Gamma component is analyzed using two different analytical HPLC systems. Retention time compared to E19020 α and β are given in the table below.

| | RETENTION TIME (MINUTES) | |
|---|---|---|
| COMPONENTS | SYSTEM A | SYSTEM B |
| LL-E19020α | 22.7 | 23.5 |
| LL-E19020β | 27.6 | 26.7 |
| LL-E19020γ | 18.5 | 19.6 |

A. HPLC system: Alltech adsorbosphere HS 5µ C18 column (4.6X250 mm) with guard column, eluted with a gradient of acetonitrile in 1% aqueous acetic acid. The starting composition is 40% acetonitrile linearly increasing to 70% over 25 minutes and holding at 70% for 5 minutes. The flow rate is 1.0 mL per minute.

B. HPLC system: Alltech adsorbosphere HS 5µ C18 (4.6X250 mm) with guard column, eluted with a gradient of dioxane in 1% aqueous acetic acid. The starting composition is 55% dioxane, increasing to 70% over 25 minutes and holding at 70% for 5 minutes. The flow rate is 1.0 mL per minutes.

### EXAMPLE 5

### In Vitro Antibacterial Activity Of LL-E19020 Gamma

The in vitro antibacterial activity of LL-E19020 Gamma is determined against a spectrum of gram positive and gram negative bacteria by a standard agar dilution method. Mueller-Hinton agar containing 5% sheep blood and two-fold decreasing concentrations of LL-E19020 Gamma is poured into petri dishes. The agar surfaces are inoculated with 1 to 5X10⁴ colony forming units of bacteria by means of the Steers replicating device. The lowest concentration of antibiotic that inhibits growth of a bacterial strain after 18 hours incubation is recorded as the minimal inhibitory concentration for that strain.

### Minimum Inhibitory Concentration Procedure By Agar Dilution

1. Serial two-flow dilutions of drug are prepared in Mueller-Hinton broth in a range of 2560 µg/ml-0.15 µg/ml plus a solvent control.
2. Two milliliters of drug dilution (10X) are added to sterile screw cap bottles to which 18 ml of Mueller-Hinton agar containing 5.6% defibrinated sheep blood is added. Final drug concentration ranges 256 µg/ml-0.015 µg/ml in agar containing 5% sheep blood.
3. A few isolated colonies of each test organism are inoculated into 5 ml trypticase soy broth or brain heart infusion broth. The cultures are shaken at 35^{o}C. for 5 hours.
4. Each culture is diluted 1:50 (10^{-1.7}) in Mueller-Hinton broth and applied to agar plates using a Steers replicator. Control plates should be seeded last to ensure that viable organisms are present throughout the procedure. Inoculated agar plates are allowed to stand undisturbed until the inoculum spots are completely absorbed.
5. The plates are inverted and incubated at 35^{o}C. for 18 hours with CO₂.
6. The minimum inhibitory concentration (MIC) is taken as the lowest concentration of antimicrobial agent at which complete inhibition of antimicrobial agent at which complete inhibition occurs. A very fine, barely visible haze or a single colony is disregarded.

The results are as follows:

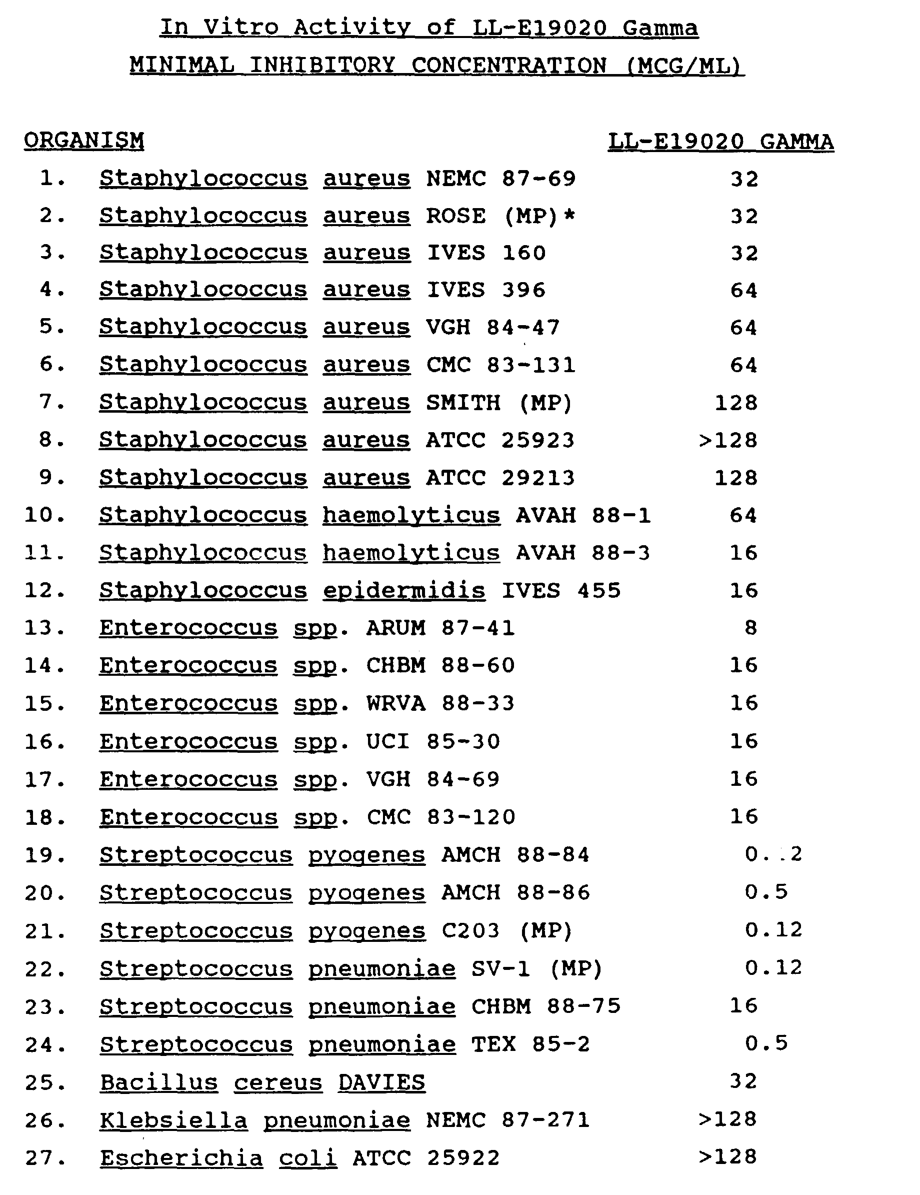

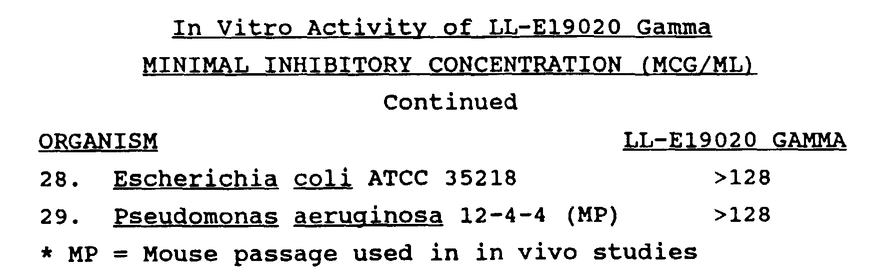

As can be seen from the in vitro data above, LL-E19020 Gamma demonstrated no gram-negative activity (MIC >128 µg/ml), showed poor to moderate activity vs staphylococci and enterococci (MIC 8-128 µg/ml) and relatively good activity against non-enterococcal streptococci (MIC 0.12-16 µg/ml).

### EXAMPLE 6

### In Vivo Activity Of LL-E19020 Gamma

The in vivo antibacterial activity of antibiotic LL-E19020 Gamma is established by infecting female CD-1 mice from Charles River Laboratories, weighing 20±2 g each, intraperitoneally with either 2.0 X 10² CFU/0.5 ml Streptococcus pyogenes C203 suspended in broth or 2.8 X 10⁶ CFU/0.5 ml Straphylococcus aureus Smith suspended in 5% hog gastric mucin. The mice were treated subcutaneously, 30 minutes after infection with the indicated dose of the test compound in 0.5 ml of 0.2% aqueous agar. Toxicity studies were performed administering the same treatment to uninfected mice.

The results are as follows:

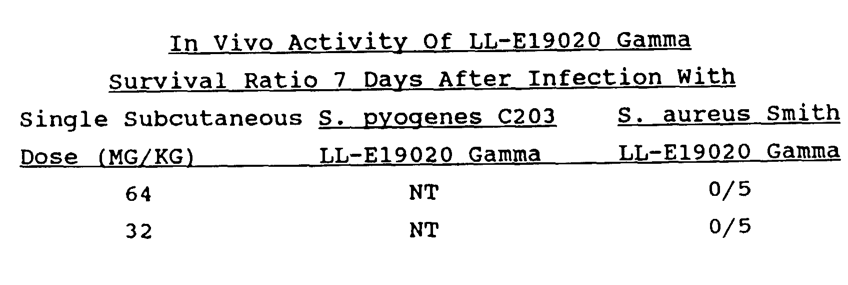

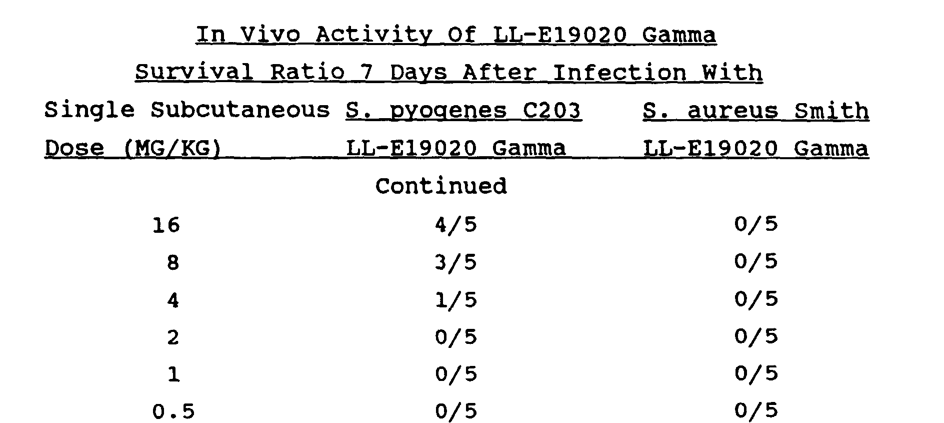

| In Vivo Toxicity Of LL-E19020 Gamma Survival Ratio 7 Days After Administration Of Single Subcutaneous | |
|---|---|
| Dose (MG/KG) | LL-E19020 Gamma |
| 64 | 2/2 |
| 32 | 2/2 |
| 16 | 2/2 |
| 8 | 2/2 |
| 4 | 2/2 |
| 2 | 2/2 |
| 1 | 2/2 |
| 0.5 | 2/2 |

The data above shows the in vivo activity of LL-E19020 gamma vs acute lethal infections in mice. LL-E19020 gamma had an approximate ED₅₀ between 4-8 mg/kg/ssc vs Streptococcus pyogenes C203. Against the Staphylococcus aureus Smith infection LL-E19020 gamma had an approximate ED₅₀ of >64 mg/kg/ssc. LL-E19020 gamma did not exhibit toxic symptoms when dosed subcutaneously at the same levels used in the protection study.

Antibiotic LL-E19020 Gamma derives its utility from antibacterial activity. For example this antibiotic may be used in the suppression of bacterial infections, as a topical antibacterial agent and as a general disinfectant for laboratories. In addition to its antibacterial activity this compound is effective as an anticoccidial agent in poultry and as a growth promotant and anthelmintic agent.

In therapeutic use, the compound of this invention may be administered in the form of a conventional pharmaceutical composition appropriate for the intended use. Such a composition may be formulated so as to be suitable for oral, parenteral, or topical administration. The active ingredient may be combined in admixture with a nontoxic pharmaceutically acceptable carrier, which carrier may take a wide variety of forms depending on the form of preparation desired for administration, ie, oral, parenteral or topical.

When the compound is employed for the above utility, it can be combined with one or more pharmaceutically acceptable carriers, for example, solvents, diluents and the like, and may be administered orally in such forms as tablets, capsules, dispersible powders, granules, or suspensions containing, for example, from about 0.05 to 5% of suspending agent, syrups containing, for example from about 10 to 50% of sugar, and elixirs containing, for example, from about 20 to 50% ethanol, and the like, or parenterally in the form of sterile injectable solutions or suspensions containing from about 0.05 to 5% suspending agent in an isotonic medium. Such pharmaceutical preparations may contain, for example, from about 0.05 up to about 90% of the active ingredient in combination with the carrier, more usually between about 5% and 60% by weight.

An effective amount of compound from 0.2 mg/kg of body weight to 100.0 mg/kg of body weight should be administered one to five times per day via any topical route of administration including but not limited to oral, parenteral (including subcutaneous, intravenous, intramuscular, intrasternal injection or infusion techniques), by inhalation spray, or rectally, in dosage unit formulations containing conventional non-toxic pharmaceutically acceptable carriers, adjuvants and vehicles. It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

These active compounds may be administered orally as well as by intravenous, intramuscular, or subcutaneous routes. Solid carriers include starch, lactose, dicalcium phosphate, microcrystalline cellulose, sucrose and kaolin, while liquid carriers include sterile water, polyethylene glycols, non-ionic surfactants and edible oils such as corn, peanut and sesame oils, as are appropriate to the nature of the active ingredient and the particular form of administration desired. Adjuvants customarily employed in the preparation of pharmaceutical compositions may be advantageously included, such as flavoring agents, coloring agents, preserving agents, and antioxidants, for example, vitamin E, ascorbic acid, BHT and BHA.

The preferred pharmaceutical compositions from the stand-point of ease of preparation and administration are solid compositions, particularly tablets and hard-filled or liquid-filled capsules. Oral administration of the compound is preferred.

These active compounds may also be administered parenterally or intraperitoneally. Solutions or suspensions of these active compounds as a free base or pharmacologically acceptable salt can be prepared in water suitably mixed with a surfactant such as hydroxy propylcellulose. Dispersions can also be prepared in glycerol, liquid polyethylene glycols and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), suitable mixtures thereof, and vegetable oil.

The present invention relates to a method for increasing the growth rate of meat-producing animals and fish and for increasing the efficiency of feed utilization thereby by administering to the animals or fish an effective amount of an antibiotic selected from the group consisting of LL-E19020 gamma and the pharmacologically acceptable salts thereof.

This invention also relates to a method for preventing, ameliorating or controlling bacterial infections in warm-blooded animals by administering to the animals a therapeutically effective amount of an antibiotic selected from the group consisting of LL-E19020 gamma and the pharmacologically suitable salts thereof.

The present invention further relates to animal feed compositions suitable for oral administration and effective for the enhancement of performance in meat-producing animals and fish and for the prevention and control of bacterial infection in meat-producing and companion animals.

In accordance with the invention, the antibiotic compound LL-E19020 gamma or a pharmacologically suitable salt thereof may be orally or parenterally administered to both monogastric and ruminant animals. The compound may be administered in admixture with the animal's feed or as a top dressing therefor. It may also be administered to the animal in the form of a bolus, pellet, tablet, pill, oral gel or the like or provided in the animal's drinking water.

When orally administered in or with the feed, generally a total concentration of 0.001 ppm to about 1,000 ppm of the antibiotic LL-E19020 gamma or a pharmacologically acceptable salt thereof is effective for enhancing the growth rate and improving the efficiency of feed utilization by the host animal.

It is understood that since the antibiotics of the present invention are useful in the treatment of both monogastric and ruminant animals which may range in weight from only a few grams to as much as several thousand kilograms, the effective level of antibiotic required for treatment will vary. Further, effective levels for each animal will vary with the animal's stage of development and from species to species.

Compounds of this invention are particularly effective for inducing weight gain and improving feed efficiency in cattle, sheep, swine, goats, rabbits, horses and poultry.

Animal feed compositions which will provide the desired growth enhancement and feed efficiency in the meat-producing animal may be prepared by admixing the above-said antibiotic or salt thereof, or an animal feed supplement containing same, with a sufficient quantity of an appropriate animal feed to provide the desired level of active compound in the finished feed.

Animal feed supplements may be prepared by admixing about 1.0% to 75% by weight of the antibiotic or salt thereof, with about 99% to 25% by weight of carriers or diluents. Carriers or diluents suitable for use in the preparation of the feed supplements include the following: alfalfa meal, soybean meal, cottonseed oil meal, linseed oil meal, sodium chloride, corn meal, cane molasses, urea, bone meal, fish meal, corncob meal, calcium chloride, and other similar materials. Use of the carriers or diluents in feed supplements promote uniformity of distribution of the active ingredient in the finished feed into which the supplement is blended. It thus performs an important function by ensuring proper distribution of the active ingredient throughout the feed.

In actual agronomic practice, the supplement may be used as a top dressing to help ensure uniformity of distribution of the active compound across the top of the dressed feed.

For parenteral administration, the antibiotic or antibiotic salt may be prepared in the form of a paste or pellet and administered as an implant, usually under the skin of the head or ear of the animal in which enhanced growth rate and/or improved efficiency of feed utilization is desired.

In practice, parenteral administration generally involves injection of a sufficient amount of the above-said antibiotic, or antibiotic salt, to provide the animal with from about 0.01 to 100 mg/kg of body weight per day of the active ingredient.

Paste formulations may be prepared by dispersing the antibiotic or antibiotic salt in a pharmaceutically acceptable oil, such as, for example, peanut oil, sesame oil and corn oil.

Pellets containing an effective level of the antibiotic selected from LL-E19020 gamma may be prepared by admixing the antibiotic with a diluent, such as carbowax, biodegradable polymers, carnauba wax, or the like. A lubricant, such as magnesium stearate or calcium stearate, may be added to improve the pelleting process, if desired.

It is, of course, recognized that more than one pellet may be administered to an animal to achieve the desired dose level which will provide the increased growth rate and/or improve efficiency of feed utilization by said animal. Moreover, it has been found that additional implants may also be introduced periodically during the treatment period in order to maintain the proper drug release rate in the animal's body.

Advantageously, parenteral or oral administration of the antibiotic compounds of the present invention prevents, controls and ameliorates bacterial disease common to the current methods of livestock production. Among such diseases is swine dysentery, also known as bloody scours and hemorrhagic colitis, and which is frequently encountered in swine husbandry. This widespread disease is generally characterized by one or more of the following symptoms: diarrhea, hemorrhagic diarrhea, stunted growth, staggering gait, swelling of the eyelids and coarseness of the hair. Another important agronomic disease is necrotic enteritis, a severe intestinal disease encountered in poultry production. Both swine dysentery and necrotic enteritis, when left unchecked, have a significant economic impact on livestock production.

In accordance with this invention, for prophylactic administration, the antibiotic selected from LL-E19020 gamma or a pharmacologically suitable salt is intimately mixed in the feed ration or drinking water of the infected swine or poultry. The antibiotic may also be suitably prepared as a premix or feed supplement as described hereinabove.

### EXAMPLE 7

### Evaluation of test compound for increasing the growth of chickens and improving the efficiency of feed utilization thereby

In this test, one day old Peterson X Arbor Acres chicks are sorted into equal weight groups of 5 males and 5 females per cage. Cages are randomized to treatment groups with six replicates per treatment. The test compound is evaluated 25 ppm in the diet against chicks receiving a non-medicated diet and a diet containing 200 ppm of penicillin as a positive control.

The cages are weighed at day 1 and at day 14 and feed consumption is measured on weigh days. Feed and water are offered *ad libitum* and lighting and supplemental heat are provided continuously.

The poultry diet employed in the test is as follows:

| | |
|---|---|
| Vitamin-amino acid premix | 0.5% |
| Trace minerals | 0.1% |
| Sodium chloride | 0.3% |
| Dicalcium phosphate | 1.2% |
| Ground limestone | 0.5% |
| Stabilized fat | 4.0% |
| Dehydrated alfalfa, 17% protein | 2.0% |
| Corn gluten meal, 41% protein | 5.0% |
| Menhaden fish meal, 60% protein | 5.0% |
| Soybean oil meal, 44% protein | 30.0% |
| Ground yellow corn | 100.0% |

The vitamin-amino acid premix in the above feed composition is prepared from the following formulation. The expressions of quantity relate to units per kilogram of the finished feed composition.

| | |
|---|---|
| Butylated hydroxy toluene | 125.0 mg |
| dl-Methionine | 500.0 mg |
| Vitamin A | 3300.0 I.U. |
| Vitamin D₃ | 1100.0 I.C.U. |
| Riboflavin | 4.4 mg |
| Vitamin E | 2.2 I.U. |
| Niacin | 27.5 mg |
| Panthothenic acid | 8.8 mg |
| Choline chloride | 500.0 mg |
| Folic acid | 1.43 mg |
| Menadione sodium bisulfate | 1.1 mg |
| Vitamin B₁₂ | 11.0 mcg |
| Ground yellow corn | 5.0 mg |

Data obtained are reported in Table II below where it can be seen that antibiotic LL-E19020γ both improved weight gain of chicks and increased the efficiency of feed utilization thereby over unmedicated controls.

**Table II**

| Treatment | ppm | Weight Gain (g) | % improvement over control | feed conversion | % improvement over control |
|---|---|---|---|---|---|
| Control | - | 321 | - | 1.322 | - |
| E19020 gamma | 25 | 342 | 6.5 | 1.297 | 1.9 |
| Penicillin | 200 | 344 | 7.2 | 1.317 | 0.4 |

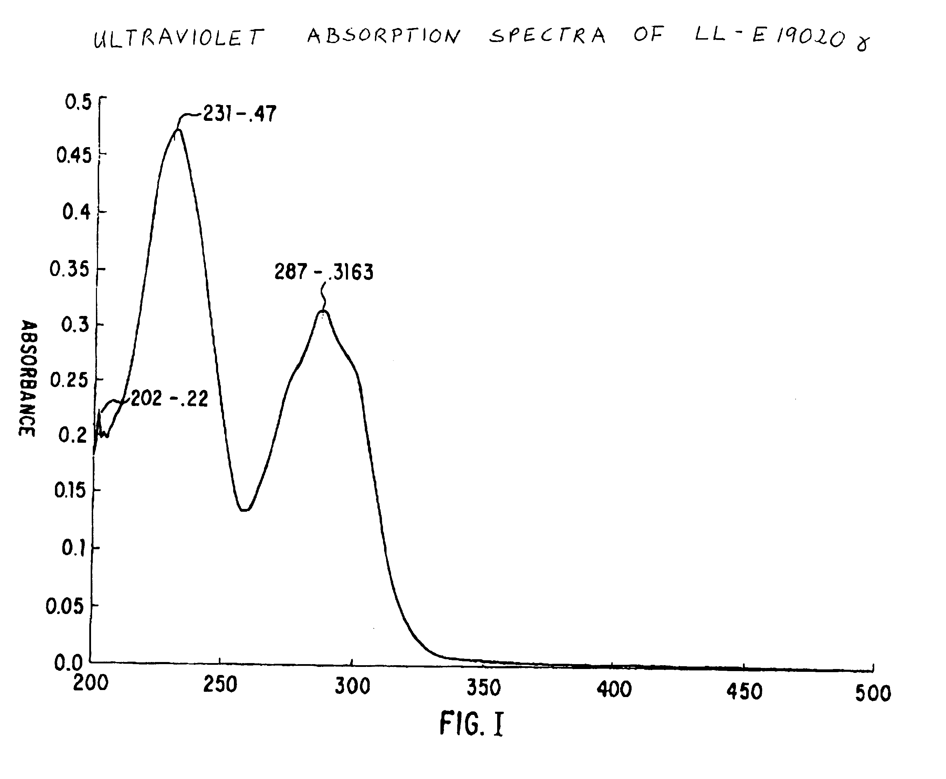

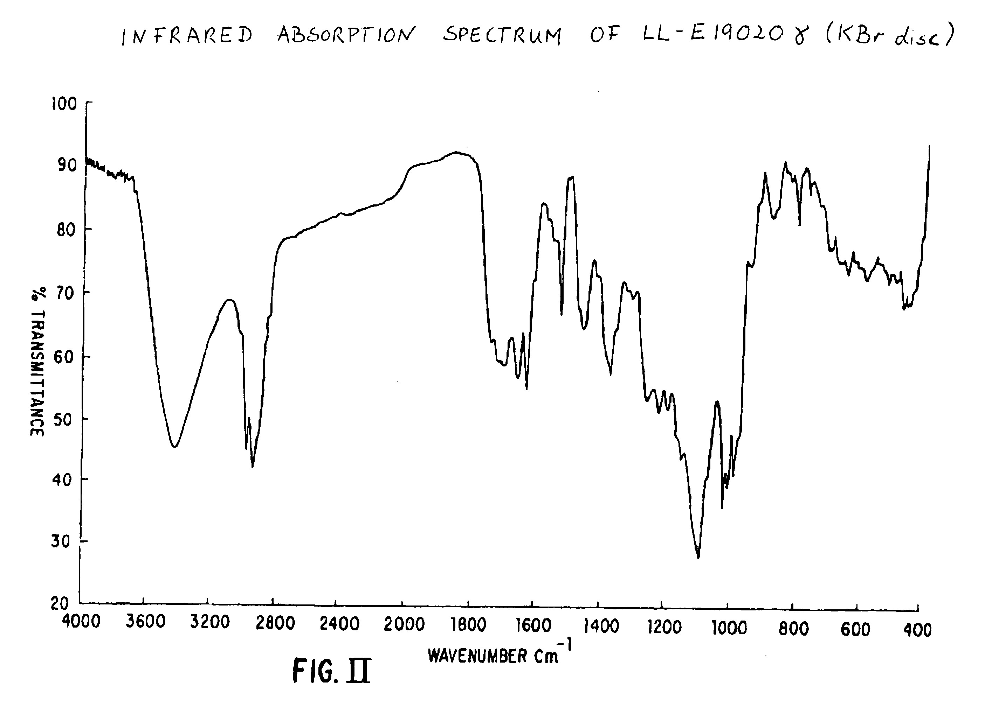

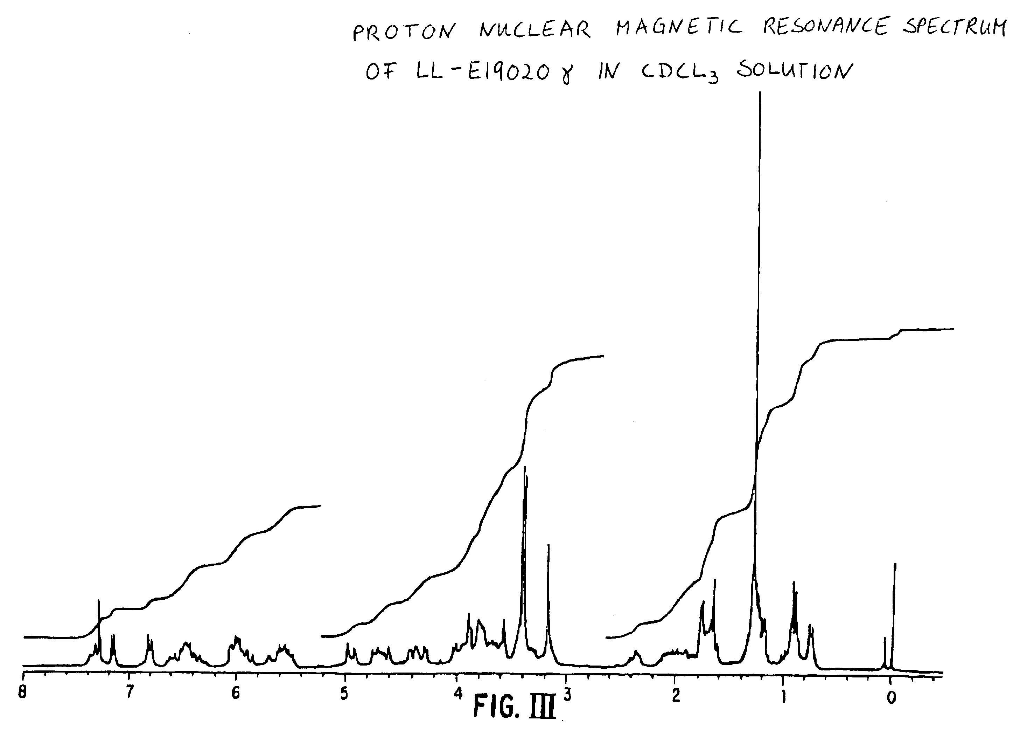

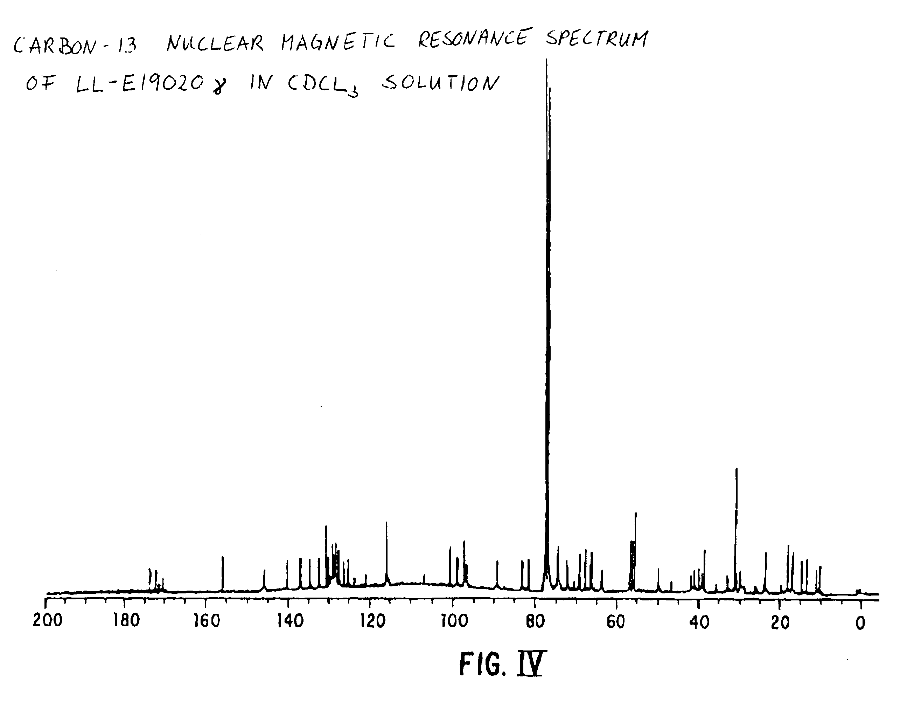

## Claims (Claims for the following Contracting State(s): AT, BE, CH, LI, DE, DK, FR, GB, IT, LU, NL, PT, SE)

1. A compound LL-E19020 Gamma comprising
(a) the structure 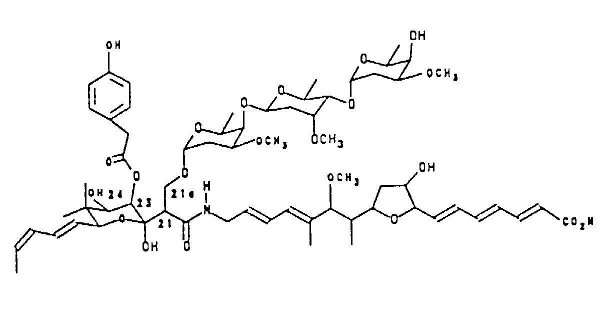
(b) an elemental analysis: C 62.22; H 7.77; N 0.92;
(c) a molecular weight of 1241 (FABMS = M/Z 1264 corresponding to [M+Na]+);
(d) a specific optical rotation:
[α] _{D}^{26°} = -7°(1.001, MeOH)
(e) a characteristic ultraviolet absorption spectrum as shown in Figure I of the attached drawings;
(f) a characteristic infrared absorption spectrum as shown in Figure II of the attached drawings;
(g) a characteristic proton nuclear magnetic resonance spectrum as shown in Figure III of the attached drawings;
(h) a characteristic carbon-13 nuclear magnetic resonance spectrum as shown in Figure IV of the attached drawings.
(i) a characteristic HPLC retention time of 18.5 minutes using a gradient of acetonitrile in aqueous acetic acid; and
(j) a characteristic HPLC retention time of 19.6 minutes using a gradient of dioxane in aqueous acetic acid.

2. A process for producing antibiotic LL-E19020 Gamma as defined in Claim 1 which comprises aerobically fermenting the organism Streptomyces lydicus ssp. tanzanius NRRL 18036 or mutants thereof in a liquid medium containing assimilable sources of carbon, nitrogen and inorganic salts, until substantial antibiotic activity is imparted to said medium and then recovering the antibiotic LL-E19020 Gamma therefrom.

3. A process for producing antibiotic LL-E19020 Gamma as defined in Claim 1 which comprises aerobically fermenting a liquid medium containing assimilable sources of carbon, nitrogen and inorganic salts, which medium has been inoculated with a viable culture of the organism Streptomyces lydicus ssp. tanzanius NRRL 18036 or mutants thereof, maintaining said fermentation culture at a temperature of 25-32°C for a period of about 80-200 hours, harvesting the mash and extracting the antibiotic.

4. The compound LL-E19020 Gamma as defined in Claim 1 for treating bacterial infections in warm-blooded animals.

5. A antibiotic pharmaceutical composition which comprises an antibiotic amount of LL-E19020 Gamma as defined in Claim 1 in association with a pharmaceutically acceptable carrier.

6. Use of an antibiotic selected from the group consisting of LL-E19020 Gamma as defined in Claim 1 or a pharmacologically suitable salt thereof for the preparation of a medicine for increasing the growth rate of meat-producing animal and fish.

7. The use according to Claim 6, wherein the antibiotic or antibiotic salt is orally administered to the meat-producing animals or fish in the feed or water at a concentration of about 0.001 ppm to 1,000 ppm.

8. The use according to Claim 6, wherein the antibiotic or antibiotic salt is parenterally administered to the meat-producing animals at a dose rate sufficient to provide about 0.01-100 mg per kg of body weight per day.

9. Use of an antibiotic selected from the group consisting of LL-E19020 Gamma as defined in Claim 1 or a pharmacologically suitable salt thereof for the preparation of a medicine for oral administration for increasing the efficiency of food utilization by meat-producing animals.

10. The use according to Claim 9 wherein the antibiotic or antibiotic salt is administered in the feed or water at a concentration of about 0.001 ppm to 1,000 ppm.

11. An animal feed composition for increasing the growth rate of meat-producing animals and fish comprising an edible carrier and a growth rate increasing amount of an antibiotic selected from the group consisting of LL-E19020 gamma, as defined in Claim 1 or a pharmacologically suitable salt thereof.

12. The animal feed composition according to Claim 11 wherein the antibiotic is present in an amount sufficient to provide a final concentration of about 0.001-1,000 ppm of antibiotic in the feed.

13. An animal feed composition for increasing the efficiency of food utilization comprising an edible carrier and a feed efficiency increasing amount of an antibiotic selected from the group consisting of LL-E19020 gamma, as defined in Claim 1 or a pharmacologically suitable salt thereof.

14. An animal feed composition for preventing, ameliorating or controlling bacterial infection in warm-blooded animals comprising an edible carrier and an antibacterially effective amount of an antibiotic selected from the group consisting of LL-E19020 gamma, as defined in Cliam 1 or a pharmacologically suitable salt thereof.

## Claims (Claims for the following Contracting State(s): ES, GR)

1. A process for producing antibiotic LL-E19020 Gamma comprising
(a) the structure 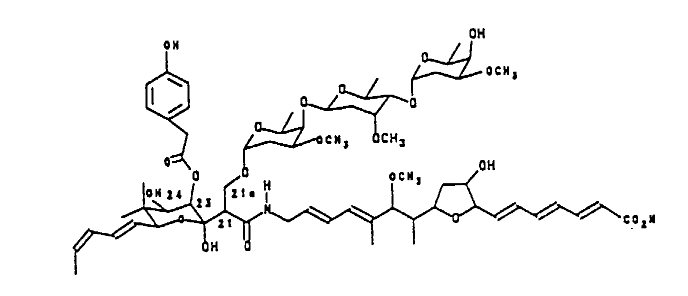
(b) an elemental analysis: C 62.22; H 7.77; N 0.92;
(c) a molecular weight of 1241 (FABMS = M/Z 1264 corresponding to [M+Na]+);
(d) a specific optical rotation:
[α] _{D}^{26°} = -7°(1.001, MeOH)
(e) a characteristic ultraviolet absorption spectrum as shown in Figure I of the attached drawings;
(f) a characteristic infrared absorption spectrum as shown in Figure II of the attached drawings;
(g) a characteristic proton nuclear magnetic resonance spectrum as shown in Figure III of the attached drawings;
(h) a characteristic carbon-13 nuclear magnetic resonance spectrum as shown in Figure IV of the attached drawings.
(i) a characteristic HPLC retention time of 18.5 minutes using a gradient of acetonitrile in aqueous acetic acid; and
(j) a characteristic HPLC retention time of 19.6 minutes using a gradient of dioxane in aqueous acetic acid,
which comprises aerobically fermenting the organism Streptomyces lydicus ssp. tanzanius NRRL 18036 or mutants thereof in a liquid medium containing assimilable sources of carbon, nitrogen and inorganic salts, until substantial antibiotic activity is imparted to said medium and then recovering the antibiotic LL-E19020 Gamma therefrom.

2. A process for producing antibiotic LL-E19020 Gamma comprising
(a) the structure 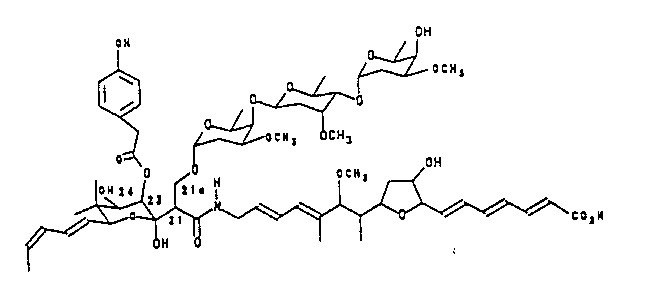
(b) an elemental analysis: C 62.22; H 7.77; N 0.92;
(c) a molecular weight of 1241 (FABMS = M/Z 1264 corresponding to [M+Na]+);
(d) a specific optical rotation:
[α] _{D}^{26°} = -7°(1.001, MeOH)
(e) a characteristic ultraviolet absorption spectrum as shown in Figure I of the attached drawings;
(f) a characteristic infrared absorption spectrum as shown in Figure II of the attached drawings;
(g) a characteristic proton nuclear magnetic resonance spectrum as shown in Figure III of the attached drawings;
(h) a characteristic carbon-13 nuclear magnetic resonance spectrum as shown in Figure IV of the attached drawings.
(i) a characteristic HPLC retention time of 18.5 minutes using a gradient of acetonitrile in aqueous acetic acid; and
(j) a characteristic HPLC retention time of 19.6 minutes using a gradient of dioxane in aqueous acetic acid,
which comprises aerobically fermenting a liquid medium containing assimilable sources of carbon, nitrogen and inorganic salts, which medium has been inoculated with a viable culture of the organism Streptomyces lydicus ssp. tanzanius NRRL 18036 or mutants thereof, maintaining said fermentation culture at a temperature of 25-32°C for a period of about 80-200 hours, harvesting the mash and extracting the antibiotic.

3. Use of the compound LL-E19020 Gamma obtainable according to claims 1 or 2 for the preparation of a medicine for treating bacterial infections in warm-blooded animals.

4. A process for producing an antibiotic pharmaceutical composition which comprises formulating an antibiotic amount of LL-E19020 Gamma obtainable according to claims 1 or 2 in association with a pharmaceutically acceptable carrier.

5. Use of an antibiotic selected from the group consisting of LL-E19020 Gamma obtainable according to claims 1 or 2 or a pharmacologically suitable salt thereof for the preparation of a medicine for increasing the growth rate of meat-producing animal and fish.

6. The use according to Claim 5, wherein the antibiotic or antibiotic salt is orally administered to the meat-producing animals or fish in the feed or water at a concentration of about 0.001 ppm to 1,000 ppm.

7. The use according to Claim 5, wherein the antibiotic or antibiotic salt is parenterally administered to the meat-producing animals at a dose rate sufficient to provide about 0.01-100 mg per kg of body weight per day.

8. Use of an antibiotic selected from the group consisting of LL-E19020 Gamma obtainable according to claims 1 or 2 or a pharmacologically suitable salt thereof for the preparation of a medicine for oral administation for increasing the efficiency of food utilization by meat-producing animals.

9. The use according to Claim 8, wherein the antibiotic or antibiotic salt is administered in the feed or water at a concentration of about 0.001 ppm to 1,000 ppm.

10. A process for producing an animal feed composition for increasing the growth rate of meat-producing animals and fish comprising the step of mixing an edible carrier and a growth rate increasing amount of an antibiotic selected from the group consisting of LL-E19020 Gamma obtainable according to claims 1 or 2 or a pharmacologically suitable salt thereof.

11. The process according to Claim 10, wherein the antibiotic is added in an amount sufficient to provide a final concentration of about 0.001-1,000 ppm of antibiotic in the feed.

12. A process for producing an animal feed composition for increasing the efficiency of food utilization comprising the step of mixing an edible carrier and a feed efficiency increasing amount of an antibiotic selected from the group consisting of LL-E19020 Gamma obtainable according to claims 1 or 2 or a pharmacologically suitable salt thereof.

13. A process for producing an animal feed composition for preventing, ameliorating or controlling bacterial infection in warm-blooded animals comprising the step of mixing an edible carrier and an antibacterially effective amount of an antibiotic selected from the group consisting of LL-E19020 Gamma obtainable according to claims 1 or 2 or a pharmacologically suitable salt thereof.

## Patentansprüche (Patentansprüche für folgende(n) Vertragsstaat(en): AT, BE, CH, DE, DK, FR, GB, IT, LI, LU, NL, PT, SE)

1. Verbindung LL-E19020 Gamma, dadurch gekennzeichnet, dass sie
(a) die Struktur 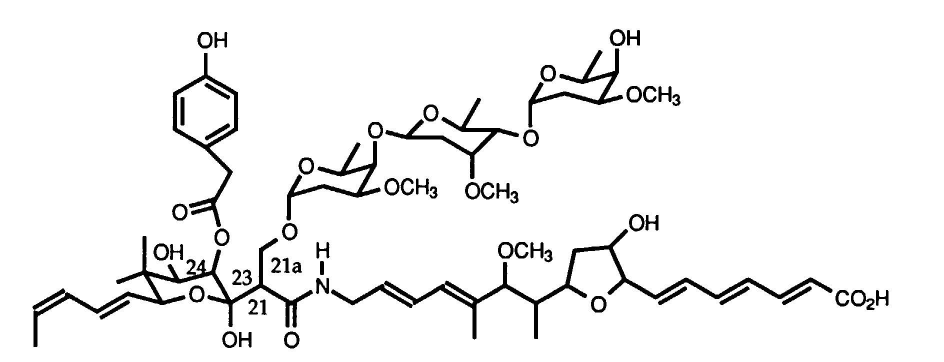
(b) eine Elementaranalyse von C 62.22; H 7.77; N 0.92;
(c) ein Molekulargewicht von 1241 (FABMS=M/Z 1264 entsprechend [M+Na]+);
d) eine spezifische optische Drehung
[α] _{D}^{26°} = -7° (1.001, MeOH);
e) ein charakteristisches Ultraviolett-Absorptionsspektrum wie in Figur I der beiliegenden Zeichnungen;
f) ein charakteristisches Infrarot-Absorptionsspektrum wie in Figur II der beiliegenden Zeichnungen;
g) ein charakteristisches Protonen-NMR-Spektrum wie in Figur III der beiliegenden Zeichnungen;
h) ein charakteristisches Kohlenstoff-13-NMR-Spektrum wie in Figur IV der beiliegenden Zeichnungen;
i) eine charakteristische HPLC-Retentionszeit von 18.5 Minuten unter Verwendung eines Eluierungsmittelgradienten von Acetonitril in wässriger Essigsäure; und
j) eine charakteristische HPLC-Retentionszeit von 19.6 Minuten unter Verwendung eines Eluierungsmittelgradienten von Dioxan in wässriger Essigsäure aufweist.

2. Verfahren zur Herstellung des in Anspruch 1 definierten Antibiotikums LL-E19020 Gamma, dadurch gekennzeichnet, dass man den Organismus Streptomyces lydicus ssp. tanzanius NRRL 18036 oder einen Mutanten davon in einem flüssigen Medium, welches assimilierbare Quellen von Kohlenstoff, Stickstoff und anorganischen Salzen enthält, aerob fermentiert bis genanntes Medium eine substantielle antibiotische Aktivität aufweist, und danach das Antibiotikum LL-E19020 Gamma daraus isoliert.

3. Verfahren zur Herstellung des in Anspruch 1 definierten Antibiotikums LL-E19020 Gamma, dadurch gekennzeichnet, dass man ein flüssiges Medium, welches assimilierbare Quellen von Kohlenstoff, Stickstoff und anorganischen Salzen enthält und welches mit einer lebensfähigen Kultur des Organismus Streptomyces lydicus ssp. tanzanius NRRL 18036 oder eines Mutanten davon inokuliert wurde, aerob fermentiert, wobei die Fermentationskultur während etwa 80-200 Stunden bei einer Temperatur von 25-32°C gehalten wird, die Maische abtrennt und das Antibiotikum extrahiert.

4. Die in Anspruch 1 definierte Verbindung LL-E19020 Gamma zur Behandlung bakterieller Infektionen in Warmblütern.

5. Eine antibiotische, pharmazeutische Komposition, dadurch gekennzeichnet, dass sie eine antibiotische Menge des in Anspruch 1 definierten LL-E19020 Gamma und einen pharmazeutisch verwendbaren Träger enthält.

6. Verwendung des in Anspruch 1 definierten Antibiotikums LL-E19020 Gamma oder eines pharmazeutisch verwendbaren Salzes davon zur Herstellung eines Medikamentes zur Erhöhung der Wachstumsrate von fleischproduzierenden Tieren und Fischen.

7. Verwendung gemäss Anspruch 6, dadurch gekennzeichnet, dass das Antibiotikum oder das Antibiotikumsalz fleischproduzierenden Tieren oder Fischen oral im Futter oder Wasser bei einer Konzentration von ungefähr 0,001 ppm bis 1000 ppm verabreicht wird.

8. Verwendung gemäss Anspruch 6, dadurch gekennzeichnet, dass das Antibiotikum oder das Antibiotikumsalz fleischproduzierenden Tieren parenteral bei einer Dosisrate verabreicht wird, welche ausreicht, ungefähr 0,01-100 mg pro kg Körpergewicht bereitzustellen.

9. Verwendung des in Anspruch 1 definierten Antibiotikums LL-E19020 Gamma oder eines pharmakologisch verwendbaren Salzes davon zur Herstellung einer Medizin zur oralen Verabreichung zwecks Erhöhung der Futterverwertung bei fleischproduzierenden Tieren.

10. Verwendung gemäss Anspruch 9, dadurch gekennzeichnet, dass das Antibiotikum oder das Antibiotikumsalz im Futter oder Wasser bei einer Konzentration von ungefähr 0,001 ppm bis 1000 ppm verabreicht wird.

11. Tierfuttermittel zur Erhöhung der Wachstumsrate von fleischproduzierenden Tieren und Fischen, dadurch gekennzeichnet, dass sie einen essbaren Träger und eine die Wachstumsrate erhöhende Menge des in Anspruch 1 definierten Antibiotikums LL-E19020 Gamma oder eines pharmakologisch verwendbaren Salzes davon enthält.

12. Tierfuttermittel gemäss Anspruch 11, dadurch gekennzeichnet, dass das Antibiotikum in einer Menge vorhanden ist, welche ausreicht, eine Konzentration von 0,001 ppm bis 1000 ppm Antibiotikum im Futter zur Verfügung zu stellen.

13. Tierfuttermittel zur Erhöhung der Futtermittelverwertung, dadurch gekennzeichnet, dass sie einen essbaren Träger und eine die Futtermittelverwertung erhöhende Menge des in Anspruch 1 definierten Antibiotikums LL-E19020 Gamma oder eines pharmakologisch verwendbaren Salzes davon enthält.

14. Tierfuttermittel zur Verhinderung, Verbesserung oder Kontrolle bakterieller Infektionen in Warmblütern, dadurch gekennzeichnet, dass sie einen essbaren Träger und eine antibakteriell wirksame Menge des in Anspruch 1 definierten Antibiotikums LL-E19020 Gamma oder eines pharmakologisch verwendbaren Salzes davon enthält.

## Patentansprüche (Patentansprüche für folgende(n) Vertragsstaat(en): ES, GR)

1. Verfahren zur Herstellung der Verbindung LL-E19020 Gamma, welche
(a) die Struktur 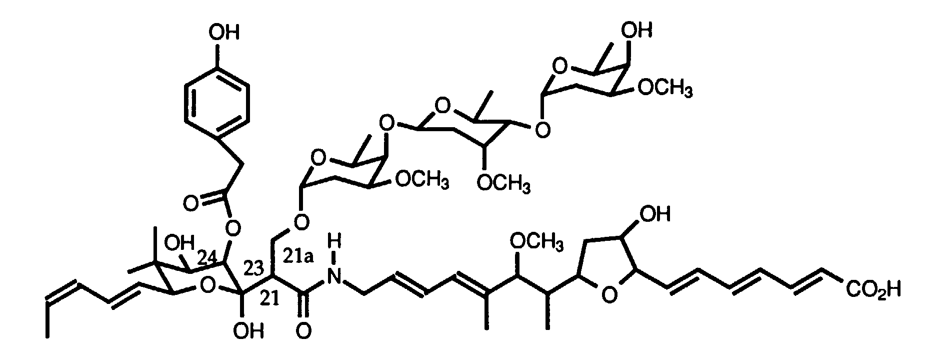
(b) eine Elementaranalyse von C 62.22; H 7.77; N 0.92;
(c) ein Molekulargewicht von 1241 (FABMS=M/Z 1264 entsprechend [M+Na]+);
d) eine spezifische optische Drehung
[α] _{D}^{26°} = -7° (1.001, MeOH);
e) ein charakteristisches Ultraviolett-Absorptions spektrum wie in Figur I der beiliegenden Zeichnungen;
f) ein charakteristisches Infrarot-Absorptionsspektrum wie in Figur II der beiliegenden Zeichnungen;
g) ein charakteristisches Protone-NMR-Spektrum wie in Figur III der beiliegenden Zeichnungen;
h) ein charakteristisches Kohlenstoff-13-NMR-Spektrum wie in Figur IV der beiliegenden Zeichnungen;
i) eine charakteristische HPLC-Retentionszeit von 18.5 Minuten unter Verwendung eines Eluierungsmittelgradienten von Acetonitril in wässriger Essigsäure; und
j) ein charakteristische HPLC-Retentionszeit von 19.6 Minuten unter Verwendung eines Eluierungsmittelgradienten von Dioxan in wässriger Essigsäure aufweist,
dadurch gekennzeichnet, dass man den Organismus Streptomyces lydicus ssp. tanzanius NRRL 18036 oder einen Mutanten davon in einem flüssigen Medium, welches assimilierbare Quellen von Kohlenstoff, Stickstoff und anorganischen Salzen enthält aerob fermentiert bis genanntes Medium eine substantielle antibiotische Aktivität aufweist und danach das Antibiotikum LL-E19020 Gamma daraus isoliert.

2. Verfahren zur Herstellung der Verbindung LL-E19020 Gamma, welche
(a) die Struktur 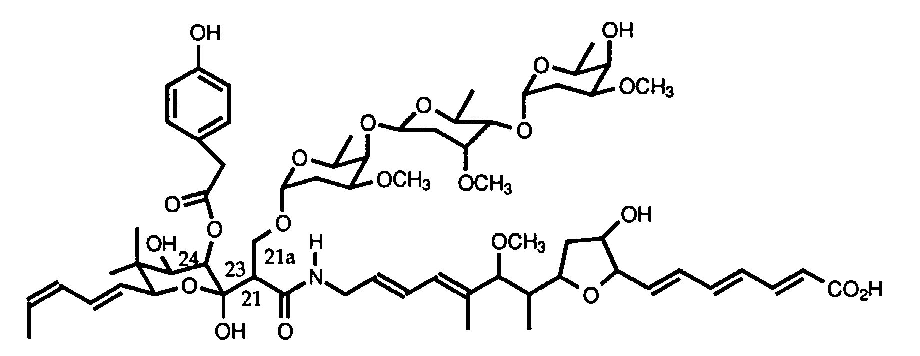
(b) eine Elementaranalyse von C 62.22; H 7.77; N 0.92;
(c) ein Molekulargewicht von 1241 (FABMS=M/Z 1264 entsprechend [M+Na]+);
d) eine spezifische optische Drehung
[α] _{D}^{26°} = -7° (1.001, MeOH);
e) ein charakteristisches Ultraviolett-Absorptions spektrum wie in Figur I der beiliegenden Zeichnungen;
f) ein charakteristisches Infrarot-Absorptionsspektrum wie in Figur II der beiliegenden Zeichnungen;
g) ein charakteristisches Protone-NMR-Spektrum wie in Figur III der beiliegenden Zeichnungen;
h) ein charakteristisches Kohlenstoff-13-NMR-Spektrum wie in Figur IV der beiliegenden Zeichnungen;
i) eine charakteristische HPLC-Retentionszeit von 18.5 Minuten unter Verwendung eines Eluierungsmittelgradienten von Acetonitril in wässriger Essigsäure; und
j) ein charakteristische HPLC-Retentionszeit von 19.6 Minuten unter Verwendung eines Eluierungsmittelgradienten von Dioxan in wässriger Essigsäure aufweist,
dadurch gekennzeichnet, dass man ein flüssiges Medium, welches assimilierbare Quellen von Kohlenstoff, Stickstoff und anorganischen Salzen enthält und welches mit einer lebensfähigen Kultur des Organismus Streptomyces lydicus ssp. tanzanius NRRL 18036 oder eines Mutanten davon inokuliert wurde, aerob fermentiert, wobei die Fermentationskultur während etwa 80-200 Stunden bei einer Temperatur von 25-32°C gehalten wird, die Maische abtrennt und das Antibiotikum extrahiert.

3. Verwendung der gemäss Anspruch 1 und 2 erhältlichen Verbindung LL-E19020 Gamma zur Behandlung bakterieller Infektionen in Warmblütern.

4. Verfahren zur Herstellung einer antibiotischen, pharmazeutischen Komposition, dadurch gekennzeichnet, dass man eine antibiotische Menge des gemäss Anspruch 1 und 2 erhältlichen LL-E19020 Gamma und einen pharmazeutisch verwendbaren Träger formuliert.

5. Verwendung des gemäss Anspruch 1 und 2 erhältlichen Antibiotikums LL-E19020 Gamma oder eines pharmazeutisch verwendbaren Salzes davon zur Herstellung eines Medikamentes zur Erhöhung der Wachstumsrate von fleischproduzierenden Tieren und Fischen.

6. Verwendung gemäss Anspruch 5, dadurch gekennzeichnet, dass das Antibiotikum oder das Antibiotikumsalz fleischproduzierenden Tieren oder Fischen oral im Futter oder Wasser bei einer Konzentration von ungefähr 0,001 ppm bis 1000 ppm verabreicht wird.

7. Verwendung gemäss Anspruch 5, dadurch gekennzeichnet, dass das Antibiotikum oder das Antibiotikumsalz fleischproduzierenden Tieren parenteral bei einer Dosisrate verabreicht wird, welche ausreicht, ungefähr 0,01-100 mg pro kg Körpergewicht bereitzustellen.

8. Verwendung des gemäss Anspruch 1 und 2 erhältlichen Antibiotikums LL-E19020 Gamma oder eines pharmakologisch verwendbaren Salzes davon zur Herstellung einer Medizin zur oralen Verabreichung zwecks Erhöhung der Futterverwertung bei fleischproduzierenden Tieren.

9. Verwendung gemäss Anspruch 8, dadurch gekennzeichnet, dass das Antibiotikum oder das Antibiotikumsalz im Futter oder Wasser bei einer Konzentration von ungefähr 0,001 ppm bis 1000 ppm verabreicht wird.

10. Verfahren zur Herstellung eines Tierfuttermittels zur Erhöhung der Wachstumsrate von fleischproduzierenden Tieren und Fischen, dadurch gekennzeichnet, dass man einen essbaren Träger und eine die Wachstumsrate erhöhende Menge des gemäss Anspruch 1 und 2 erhältlichen Antibiotikums LL-E19020 Gamma oder eines pharmakologisch verwendbaren Salzes vermischt.

11. Verfahren gemäss Anspruch 10, dadurch gekennzeichnet, dass das Antibiotikum in einer Menge zugegeben wird, welche ausreicht, eine Konzentration von 0,001 ppm bis 1000 ppm Antibiotikum im Futter bereitzustellen.

12. Verfahren zur Herstellung eines Tierfuttermittels zur Erhöhung der Futtermittelverwertung, dadurch gekennzeichnet, dass man einen essbaren Träger und eine die Futtermittelverwertung erhöhende Menge des gemäss Anspruch 1 und 2 erhältlichen Antibiotikums LL-E19020 Gamma oder eines pharmakologisch verwendbaren Salzes davon vermischt.

13. Verfahren zur Herstellung eines Tierfuttermittels zur Verhinderung, Verbesserung oder Kontrolle bakterieller Infektionen in Warmblütern, dadurch gekennzeichnet, dass man einen essbaren Träger und eine antibakteriell wirksame Menge des gemäss Anspruch 1 und 2 erhältlichen Antibiotikums LL-E19020 Gamma oder eines pharmakologisch verwendbaren Salzes davon vermischt.

## Revendications (Revendications pour l'(les) Etat(s) contractant(s) suivant(s): AT, BE, CH, DE, DK, FR, GB, IT, LI, LU, NL, PT, SE)

1. Composé LL-E19020 gamma comprenant
(a) la structure 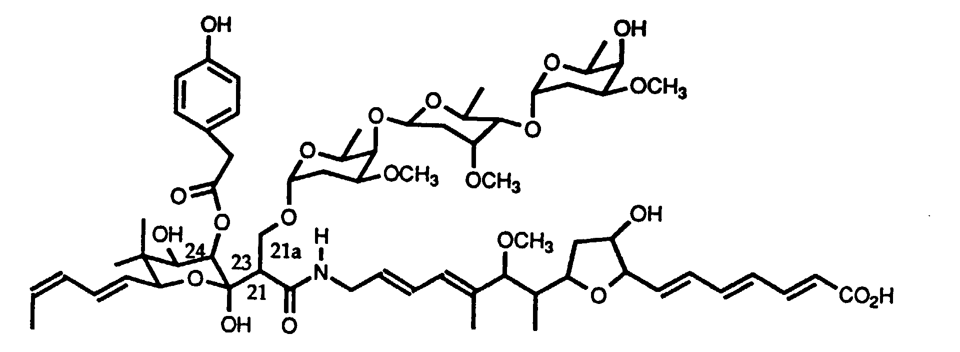
(b) une analyse élémentaire: C 62,22; H 7,77; N 0,92;
(c) une masse moléculaire de 1241 (FABMS = M/Z 1264 correspondant à [M+Na]+);
(d) une rotation optique spécifique:
[α]_{D}26° = -7° (1,001, MeOH);
(e) un spectre caractéristique d'absorption dans l'ultraviolet tel que représenté dans la figure I des dessins annexés;
(f) un spectre caractéristique d'absorption dans l'infrarouge tel que représenté dans la figure II des dessins annexés;
(g) un spectre caractéristique de résonance magnétique nucléaire du proton tel que représenté dans la figure III des dessins annexés;
(h) un spectre caractéristique de résonance magnétique nucléaire du carbone-13 tel que représenté dans la figure IV des dessins annexés;
(i) une durée caractéristique de rétention en HPLC de 18,5 minutes avec un gradient d'acétonitrile dans de l'acide acétique aqueux; et
(j) une durée caractéristique de rétention en HPLC de 19,6 minutes avec un gradient de dioxanne dans de l'acide acétique aqueux.

2. Procédé pour la production d'antibiotique LL-E19020 gamma tel que défini dans la revendication 1, qui comprend la fermentation aérobie de l'organisme Streptomyces lydicus ssp. tanzanius NRRL 18036 ou de mutants de celui-ci dans un milieu liquide contenant des sources assimilables de carbone, d'azote et de sels inorganiques, jusqu'à ce qu'une activité antibiotique substantielle soit conférée au dit milieu, et ensuite récupération de l'antibiotique LL-E19020 gamma à partir de ce milieu.

3. Procédé pour la production d'antibiotique LL-E19020 gamma tel que défini dans la revendication 1, qui comprend la fermentation aérobie d'un milieu liquide contenant des sources assimilables de carbone, d'azote et de sels inorganiques, lequel milieu a été inoculé avec une culture viable de l'organisme Streptomyces lydicus ssp. tanzanius NRRL 18036 ou de mutants de celui-ci, le maintien de ladite culture de fermentation à une température de 25-32°C pendant une durée d'environ 80-200 heures, la récolte de la bouillie et l'extraction de l'antibiotique.

4. Composé LL-E19020 gamma tel que défini dans la revendication 1 pour le traitement d'infections bactériennes chez les animaux à sang chaud.

5. Composition pharmaceutique antibiotique, qui comprend une quantité antibiotique de LL-E19020 gamma tel que défini dans la revendication 1 en association avec un support pharmaceutiquement acceptable.

6. Utilisation d'un antibiotique choisi dans le groupe consistant en LL-E19020 gamma tel que défini dans la revendication 1 ou un sel pharmacologiquement acceptable de celui-ci pour la préparation d'un médicament pour accroître le taux de croissance d'animaux producteurs de viande et de poissons.

7. Utilisation selon la revendication 6, dans laquelle l'antibiotique ou le sel d'antibiotique est administré oralement aux animaux producteurs de viande ou aux poissons dans la nourriture ou dans l'eau à une concentration d'environ 0,001 ppm à 1.000 ppm.

8. Utilisation selon la revendication 6, dans laquelle l'antibiotique ou le sel d'antibiotique est administré par voie parentérale aux animaux producteurs de viande à une dose suffisante pour fournir environ 0,01-100 mg par kg de poids corporel par jour.

9. Utilisation d'un antibiotique choisi dans le groupe consistant en LL-E19020 gamma tel que défini dans la revendication 1 ou un sel pharmacologiquement acceptable de celui-ci pour la préparation d'un médicament pour administration orale pour l'augmentation de l'efficacité de l'utilisation de la nourriture par des animaux producteurs de viande.

10. Utilisation selon la revendication 9, dans laquelle l'antibiotique ou le sel d'antibiotique est administré-dans la nourriture ou dans l'eau à une concentration d'environ 0,001 ppm à 1.000 ppm.

11. Composition de nourriture pour animaux pour augmenter le taux de croissance des animaux producteurs de viande et des poissons, comprenant un support comestible et une quantité augmentant le taux de croissance d'un antibiotique choisi dans le groupe consistant en LL-E19020 gamma, tel que défini dans la revendication 1, ou un sel pharmacologiquement acceptable de celui-ci.

12. Composition de nourriture pour animaux selon la revendication 11, dans laquelle l'antibiotique est présent en une quantité suffisante pour fournir une concentration finale d'environ 0,001-1.000 ppm d'antibiotique dans la nourriture.

13. Composition de nourriture pour animaux pour augmenter l'efficacité de l'utilisation de nourriture, comprenant un support comestible et une quantité augmentant l'efficacité de la nourriture d'un antibiotique choisi dans le groupe consistant en LL-E19020 gamma, tel que défini dans la revendication 1, ou un sel pharmacologiquement acceptable de celui-ci.

14. Composition de nourriture pour animaux pour prévenir, améliorer ou maîtriser l'infection bactérienne chez les animaux à sang chaud, comprenant un support comestible et une quantité efficace du point de vue antibactérien d'un antibiotique choisi dans le groupe consistant en LL-E19020 gamma, tel que défini dans la revendication 1, ou un sel pharmacologiquement acceptable de celui-ci.

## Revendications (Revendications pour l'(les) Etat(s) contractant(s) suivant(s): ES, GR)

1. Procédé pour la production du composé LL-E19020 gamma comprenant
(a) la structure 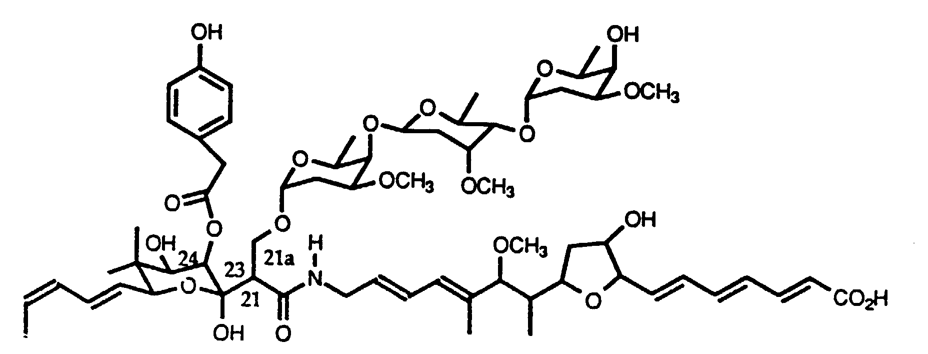
(b) une analyse élémentaire: C 62,22; H 7,77; N 0,92;
(c) une masse moléculaire de 1241 (FABMS = M/Z 1264 correspondant à [M+Na]+);
(d) une rotation optique spécifique:
[α]_{D}26° = -7° (1,001, MeOH);
(e) un spectre caractéristique d'absorption dans l'ultraviolet tel que représenté dans la figure I des dessins annexés;
(f) un spectre caractéristique d'absorption dans l'infrarouge tel que représenté dans la figure II des dessins annexés;
(g) un spectre caractéristique de résonance magnétique nucléaire du proton tel que représenté dans la figure III des dessins annexés;
(h) un spectre caractéristique de résonance magnétique nucléaire du carbone-13 tel que représenté dans la figure IV des dessins annexés;
(i) une durée caractéristique de rétention en HPLC de 18,5 minutes avec un gradient d'acétonitrile dans de l'acide acétique aqueux; et
(j) une durée caractéristique de rétention en HPLC de 19,6 minutes avec un gradient de dioxanne dans de l'acide acétique aqueux,
qui comprend la fermentation aérobie de l'organisme Streptomyces lydicus ssp. tanzanius NRRL 18036 ou de mutants de celui-ci dans un milieu liquide contenant des sources assimilables de carbone, d'azote et de sels inorganiques, jusqu'à ce qu'une activité antibiotique substantielle soit conférée au dit milieu, et ensuite récupération de l'antibiotique LL-E19020 gamma à partir de ce milieu.

2. Procédé pour la production du composé LL-E19020 gamma comprenant
(a) la structure 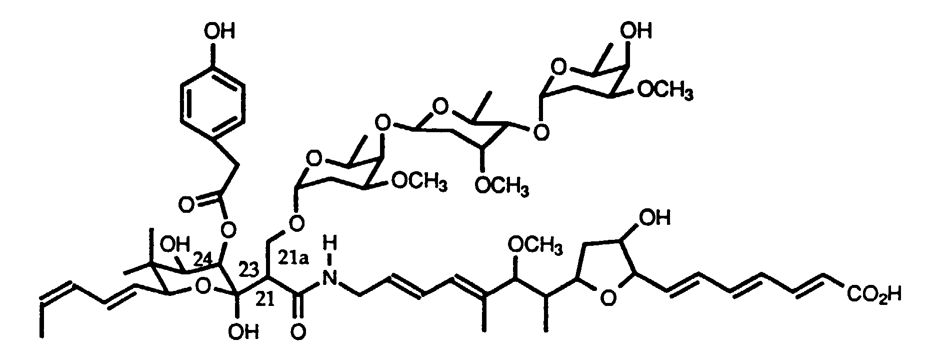
(b) une analyse élémentaire: C 62,22; H 7,77; N 0,92;
(c) une masse moléculaire de 1241 (FABMS = M/Z 1264 correspondant à [M+Na]+);
(d) une rotation optique spécifique:
[α]_{D}26° = -7° (1,001, MeOH);
(e) un spectre caractéristique d'absorption dans l'ultraviolet tel que représenté dans la figure I des dessins annexés;
(f) un spectre caractéristique d'absorption dans l'infrarouge tel que représenté dans la figure II des dessins annexés;
(g) un spectre caractéristique de résonance magnétique nucléaire du proton tel que représenté dans la figure III des dessins annexés;
(h) un spectre caractéristique de résonance magnétique nucléaire du carbone-13 tel que représenté dans la figure IV des dessins annexés;
(i) une durée caractéristique de rétention en HPLC de 18,5 minutes avec un gradient d'acétonitrile dans de l'acide acétique aqueux; et
(j) une durée caractéristique de rétention en HPLC de 19,6 minutes avec un gradient de dioxanne dans de l'acide acétique aqueux,
qui comprend la fermentation aérobie d'un milieu liquide contenant des sources assimilable de carbone, d'azote et de sels inorganiques, lequel milieu a été inoculé avec une culture viable de l'organisme Streptomyces lydicus ssp. tanzanius NRRL 18036 ou de mutants de celui-ci, le maintien de ladite culture de fermentation à une température de 25-32°C pendant une durée d'environ 80-200 heures, la récolte de la bouillie et l'extraction de l'antibiotique.

3. Utilisation du composé LL-E19020 gamma qui peut être obtenu selon les revendications 1 et 2 pour la préparation d'un médicament pour le traitement d'infections bactériennes chez les animaux à sang chaud.

4. Procédé pour la préparation d'une composition pharmaceutique antibiotique, qui comprend la formulation d'une quantité antibiotique de LL-E19020 gamma qui peut être obtenu selon les revendications 1 et 2, en association avec un support pharmaceutiquement acceptable.

5. Utilisation d'un antibiotique choisi dans le groupe consistant en LL-E19020 gamma qui peut être obtenu selon les revendications 1 et 2 ou un sel pharmacologiquement acceptable de celui-ci pour la préparation d'un médicament pour accroître le taux de croissance d'animaux producteurs de viande et de poissons.

6. Utilisation selon la revendication 5, dans laquelle l'antibiotique ou le sel d'antibiotique est administré oralement aux animaux producteurs de viande ou aux poissons dans la nourriture ou dans l'eau à une concentration d'environ 0,001 ppm à 1.000 ppm.

7. Utilisation selon la revendication 5, dans laquelle l'antibiotique ou le sel d'antibiotique est administré par voie parentérale aux animaux producteurs de viande à une dose suffisante pour fournir environ 0,01-100 mg par kg de poids corporel par jour.

8. Utilisation d'un antibiotique choisi dans le groupe consistant en LL-E19020 gamma qui peut être obtenu selon les revendications 1 et 2 ou un sel pharmacologiquement acceptable de celui-ci pour la préparation d'un médicament pour administration orale pour l'augmentation de l'efficacité de l'utilisation de la nourriture par des animaux producteurs de viande.

9. Utilisation selon la revendication 8, dans laquelle l'antibiotique ou le sel d'antibiotique est administré dans la nourriture ou dans l'eau à une concentration d'environ 0,001 ppm à 1.000 ppm.

10. Procédé pour la préparation d'une composition de nourriture pour animaux pour augmenter le taux de croissance des animaux producteurs de viande et de poissons, comprenant l'étape de mélange d'un support comestible et d'une quantité augmentant le taux de croissance d'un antibiotique choisi dans le groupe consistant en LL-E19020 gamma qui peut être obtenu selon les revendications 1 et 2, ou un sel pharmacologiquement acceptable de celui-ci.

11. Procédé selon la revendication 10, dans laquelle l'antibiotique est ajouté en une quantité suffisante pour fournir une concentation finale d'environ 0,001-1.000 ppm d'antibiotique dans la nourriture.

12. Procédé pour la préparation d'une composition de nourriture pour animaux pour augmenter l'efficacité de l'utilisation de nourriture comprenant l'étape de mélange d'un support comestible et d'une quantité augmentant l'efficacité de la nourriture d'un antibiotique choisi dans le groupe consistant en LL-E19020 gamma qui peut être obtenu selon les revendications 1 et 2, ou un sel pharmacologiquement acceptable de celui-ci.

13. Procédé pour la préparation d'une composition de nourriture pour animaux pour prévenir, améliorer ou maîtriser l'infection bactérienne chez les animaux à sang chaud, comprenant l'étape de mélange d'un support comestible et d'une quantité efficace du point de vue antibactérien d'un antibiotique choisi dans le groupe consistant en LL-E19020 gamma qui peut être obtenu selon les revendications 1 et 2, ou un sel pharmacologiquement acceptable de celui ci.
